# EUROPEAN PATENT APPLICATION

(11) **EP 2 052 740 A1**
(43) Date of publication of application: **29.04.2009**
(21) Application number: 07791996.7
(22) Date of filing: 31.07.2007
(51) Int. Cl.: A61K 47/42, A61K 38/22, B32B 9/02

(54) **CROSSLINKED GELATIN GEL MULTILAYERED STRUCTURE, CARRIER FOR BIOACTIVE FACTOR, PREPARATION FOR RELEASE OF BIOACTIVE FACTOR, AND THEIR PRODUCTION METHODS**

(30) Priority: 01.08.2006 JP 2006209855
(71) Applicant: Nichiban Co. Ltd., Tokyo 112-8663 (JP); Kyoto University, Sakyo-ku Kyoto-shi Kyoto 606-8501 (JP)
(72) Inventor: ISHIGURO, Tomoyuki, Tokyo 112-8663 (JP); FUKANO, Kenji, Tokyo 112-8663 (JP); TABATA, Yasuhiko, Kyoto-shi Kyoto 606-8507 (JP)
(74) Representative: Jones, Helen M.M.
(86) International application number: PCT/JP2007/065323
(87) International publication number: WO 2008/016163

(57) **Abstract**

A multi-layer crosslinked gelatin gel structure having a layer structure that plural layers of crosslinked gelatin gel crosslinked by irradiating gelatin or a gelatin derivative with electron beam under an oxygen-containing atmosphere are arranged adjoiningly to each other, a preparation for release of a bioactive factor with the bioactive factor contained in the multi-layer crosslinked gelatin gel structure, and production processes thereof.

## Description

### TECHNICAL FIELD

The present invention relates a multi-layer crosslinked gelatin gel structure useful as a carrier for a bioactive factor and a production process thereof. The present invention also relates to a preparation for release of a bioactive factor with the bioactive factor supported on crosslinked gelatin gel and a production process thereof. The preparation for release of the bioactive factor according to the present invention can gradually release the bioactive factor by administering it by, for example, implanting into a living body or injection using an injector.

### BACKGROUND ART

As a bioactive factor, for example, a basic fibroblast growth factor (bFGF) having a vascularization-inducing effect is known. When an aqueous solution of such a bioactive factor is administered into the living body of human or the like, the effect thereof is lost in a relatively short period of time. Many of bioactive factors are desired to sustain their effects over a relatively long period of time from the viewpoint of achieving the object of remedy. Therefore, it has been necessary to administer a large amount of the aqueous bioactive factor solution into the living body or increase the number of times of the administration.

In order to solve the above problem, there have been proposed crosslinked gelatin preparations with a bioactive factor supported on crosslinked gelatin gel. For example, U.S. Patent No. 6,831,058 B1 (hereinafter referred to as "Document 1") has proposed a crosslinked gelatin gel preparation with a basic fibroblast growth factor (bFGF) supported on crosslinked gelatin gel obtained by crosslinking alkali-treated gelatin.

Gelatin is a material high in safety for living bodies and exhibits biodegradability. Gelatin forms hydrogel and can be formed into crosslinked gelatin gel by crosslinking. The crosslinked gelatin gel forms crosslinked gelatin hydrogel in a state that water has been absorbed. The crosslinked gelatin gel also has biodegradability like gelatin, and its degradation speed in a living body can be controlled by adjusting the crosslinking density (crosslinking degree) thereof.

The crosslinked gelatin gel can support a drug having a charge, such as a bioactive factor. When the crosslinked gelatin gel, on which a bioactive factor has been supported, is administered in a living body, the bioactive factor is gradually released as the crosslinked gelatin gel is degraded in the living body. Methods for administration thereof include implanting into the living body or injection using an injector according to the form (for example, a sheet or microsphere) of the crosslinked gelatin gel.

Since a crosslinked gelatin gel preparation with a bioactive factor supported thereon can gradually release the bioactive factor in a living body, the preparation can exhibit a great effect with a small amount of the bioactive factor compared with administration in a state of an aqueous solution thereof. In particular, when the crosslinked gelatin gel with the bioactive factor supported thereon is implanted into an affected part to gradually release the bioactive factor, its effect can be locally sustained for a certain period of time with a small amount of the bioactive factor. However, conventional crosslinked gelatin gel and preparations using the crosslinked gelatin gel as a carrier involve various problems to be solved.

One of the problems is a method for crosslinking gelatin. The above Document 1 describes that methods for crosslinking gelatin include methods by a heat treatment or irradiation of ultraviolet light in addition to a method using a crosslinking agent. However, Examples of Document 1 adopt a method of crosslinking gelatin with a crosslinking agent such as glutaraldehyde or 1-ethyl-3-(3-dimethylaminopropyl)carbodiimide hydrochloride.

The method of crosslinking with the crosslinking agent requires conducting a crosslinking reaction with the crosslinking agent that is a chemical substance, and adding a reaction terminator for ending the crosslinking reaction. However, the compound used as the crosslinking agent, such as glutaraldehyde, is a toxic substance, so that there is need of taking sufficient care to secure safety so as to prevent the crosslinking agent from remaining in the crosslinked gelatin gel.

The above Document 1 describes a method of washing the crosslinked gelatin gel obtained by using the crosslinking agent with distilled water or an organic solvent such as ethanol, isopropanol or acetone. For example, Example 1 of Document 1 shows that the crosslinked gelatin gel obtained by using the crosslinking agent was washed with distilled water for 12 hours at 37°C. It takes a time as long as 24 hours to conduct the crosslinking reaction. Accordingly, the method of producing the crosslinked gelatin gel with the crosslinking agent requires a great deal of time and labor for the crosslinking reaction and the security of safety, and so there is a limit to reduction of product cost.

The method of crosslinking gelatin gel by irradiation of ultraviolet light requires initiating a crosslinking reaction with a reaction initiator and then adding a reaction terminator to deactivate the reaction initiator. However, the reaction initiator is a toxic substance, so that there is need of sufficiently wash the resultant crosslinked gelatin gel to remove the reaction initiator. Therefore, this method also requires a complicated step.

The method of crosslinking gelatin by the heat treatment requires a long time for crosslinking and is hard to control a crosslinking density, so that it is difficult to obtain crosslinked gelatin gel having a desired crosslinking degree. If a bioactive factor is supported on the crosslinked gelatin gel obtained by the heat treatment, it is thus difficult to obtain a preparation stably exhibiting desired gradual releasability.

U.S. Patent No. 5,618,312 (hereinafter referred to as "Document 2") has proposed a membrane-type medical material with a membrane material, which is obtained by impregnating a compact layer (an acellular layer of a biogenic connective tissue membrane) derived from a living body with collagen or gelatin, subjected to a physically or chemically crosslinking reaction in a field of artificial materials for living bodies, which are applied to surgical operations, such as wound prosthetic materials and burn dressing. Document 2 describes a process using physical energy such as heating, irradiation of ultraviolet light, irradiation of electron beam or irradiation of radiation as the physically crosslinking reaction. However, Examples of Document 2 only show experimental examples where crosslinking was conducted by heating. The process described in Document 2 is a process for crosslinking the membrane material obtained by impregnating the compact layer derived from the living body with collagen or gelatin, and not a production process of crosslinked gelatin gel itself.

According to the method of crosslinking gelatin by irradiation of electron beam, neither a crosslinking agent nor a reaction initiator is required, so that crosslinked gelatin gel excellent in safety for living bodies can be produced. The crosslinking method by irradiation of electron beam is relatively simple in operation and also short in time required for the crosslinking treatment.

However, in the method of crosslinking gelatin by irradiation of electron beam, there is a limit to a permeation depth of electrons into an object to be irradiated, so that this method is not always suitable for application to an object to be irradiated having a great thickness. On the other hand, the crosslinked gelatin gel used as a carrier for a bioactive factor is required to have a certain measure of thickness from the viewpoint of handling property upon implanting into a living body. In order to produce crosslinked gelatin gel uniformly crosslinked by the irradiation of electron beam, it is necessary to cause electrons to be permeated into the interior of gelatin. It is however difficult to cause the electrons to be permeated into a deep portion of the gelatin having a great thickness.

An acceleration voltage is related to a permeation depth of the electrons into the object to be irradiated, and the accelerated energy of the electrons becomes high as the acceleration voltage is made high. Therefore, an electron beam irradiation apparatus higher in acceleration voltage is required as the thickness of the object to be irradiated becomes higher. In order to uniformly crosslink gelatin gel, it is necessary to adopt a method of irradiating the gelatin gel with electron beam at such an acceleration voltage that a relative exposure dose in a depth direction of the gelatin becomes high. Therefore, large-scale equipment investment and construction of facilities having a large-scale containing capacity are required, and irradiation energy cost also becomes expensive.

The second problem involved in the prior art is that the gradual releasability of a bioactive factor cannot be controlled as the time goes on, since single-layer crosslinked gelatin gel having a substantially uniform crosslinking density is used as a carrier. It is proved that it is effective to release the bioactive factor at a high concentration in an initial stage of the administration of a crosslinked gelatin gel preparation with the bioactive factor supported thereon in a living body and then gradually release the bioactive factor at a fixed concentration over a long period of time. However, the single-layer crosslinked gelatin gel having a substantially uniform crosslinking density is degraded at a constant rate in a living body, so that change of concentration with time cannot be given in the release of the bioactive factor supported thereon.

### DISCLOSURE OF THE INVENTION

It is a problem of the present invention to provide crosslinked gelatin gel excellent in safety for living bodies and useful as a carrier for a bioactive factor and a production process thereof.

Another problem of the present invention is to provide crosslinked gelatin gel excellent in safety for living bodies and having a desired thickness and a production process thereof by irradiation of electron beam.

A further problem of the present invention is to provide crosslinked gelatin gel that can give change of concentration with time in the release of a bioactive factor supported thereon, and a production process thereof.

A still further problem of the present invention is to provide use of the crosslinked gelatin gel having such excellent various properties as described above for a carrier for a bioactive factor. More specifically, the still further problem of the present invention is to provide a preparation (crosslinked gelatin gel preparation) for release of a bioactive factor with the bioactive factor supported on the crosslinked gelatin gel having such excellent various properties as described above, and a production process thereof.

The present inventors have carried out an extensive investigation with a view toward solving the above problems. As a result, the present inventors have reached a process combining crosslinking of gelatin by irradiation of electron beam with a process of multi-layering crosslinked gelatin gel. When the crosslinked gelatin gel is multi-layered to produce a multi-layer crosslinked gelatin gel structure, the individual crosslinked gelatin gel layers can be thinned, so that a crosslinking process by irradiation of electron beam can be adopted.

Since the use of toxic substances such as a crosslinking agent and a reaction initiator is not required for the crosslinking of gelatin by the irradiation of electron beam, a crosslinked gelatin gel layer excellent in safety for living bodies can be formed. A large-scale electron beam irradiation apparatus and an excessive acceleration voltage are not always required for the irradiation of electron beam on a thinned gelatin layer. The respective crosslinked gelatin gel layers are multi-layered, whereby a multi-layer structure having a desired thickness can be obtained. A process of successively forming the respective crosslinked gelatin gel layers with them stacked on each other can be adopted for the multi-layering of the crosslinked gelatin gel layers.

On the other hand, the irradiation of electron beam is generally conducted under an atmosphere of an inert gas such as nitrogen from the viewpoints of inhibition of generation of ozone and reactivity. However, it has been proved that when the respective crosslinked gelatin gel layers are formed by irradiation of electron beam under the inert gas atmosphere, adhesion between crosslinked gelatin gel layers adjoining each other becomes poor to encounter difficulty in producing an integrated multi-layer structure.

When the multi-layer crosslinked gelatin gel structure is impregnated with an aqueous solution of a bioactive factor in a state of hydrogel or a freeze-dried state, the structure is swollen greatly compared with the original volume thereof by impregnation with the aqueous solution. At this time, interlayer separation easily occurs when the adhesion at an interface between the respective layers is poor, so that difficult is encountered on subsequent handling or administration.

When an adhesive layer is caused to intervene between the respective layers for enhancing the adhesion between the layers, a problem is offered on safety of an adhesive that is a chemical substance. In addition, a general adhesive does not exhibit biodegradability and remains in a living body.

Thus, the present inventors have carried out a further investigation. As a result, it has been found that the adhesion between the respective crosslinked gelatin gel layers adjoining each other can be sufficiently enhanced by a method of conducting irradiation of electron beam under an oxygen-containing atmosphere such as air. More specifically, an aqueous solution of gelatin is applied on to a support to form a coating layer, and the coating layer is then irradiated with electron beam under an oxygen-containing atmosphere to form a first crosslinked gelatin gel layer. The aqueous solution of gelatin is applied on to the first crosslinked gelatin gel layer to form a coating layer, and the coating layer is then irradiated with electron beam under the oxygen-containing atmosphere to form a second crosslinked gelatin gel layer. It has been found that this process is repeated desired times, thereby obtaining a multi-layer crosslinked gelatin gel structure which has a desired thickness and is excellent in adhesion between the respective crosslinked gelatin gel layers.

When exposure doses of the electron beam to the respective crosslinked gelatin gel layers are changed upon producing a multi-layer crosslinked gelatin gel structure, a multi-layer structure composed of a plurality of crosslinked gelatin gel layers different in crosslinking degree from each other can be produced.

Specifically, there is a method, in which the exposure dose of electron beam to at least one crosslinked gelatin gel layer arranged at one or both surface portions is controlled so as to become relatively smaller than the exposure dose of electron beam to at least one crosslinked gelatin gel layer arranged at other portions.

More specifically, for example, when the exposure dose to at least one crosslinked gelatin gel layer located at both surface portions (external sides) of a multi-layer crosslinked gelatin gel structure is made small, and the exposure dose to at least one crosslinked gelatin gel layer located at a core portion (internal side) is made great, the degradation speed of the crosslinked gelatin gel layer located at the external side rather than the internal side in a living body is fast, so that a multi-layer crosslinked gelatin gel structure that can release a bioactive factor supported thereon at a high concentration in an initial stage of administration and then gradually release the bioactive factor at a fixed concentration can be obtained.

As gelatin used in the present invention, may be used not only ordinary gelatin, but also a gelatin derivative such as cationized gelatin derivative or succinylated gelatin derivative.

The present invention has been led to completion on the basis of these findings.

According to the present invention, there is thus provided a multi-layer crosslinked gelatin gel structure having a layer structure that plural layers of crosslinked gelatin gel crosslinked by irradiating gelatin or a gelatin derivative with electron beam under an oxygen-containing atmosphere are arranged adjoiningly to each other.

According to the present invention, there is provided a carrier for a bioactive factor, comprising the above-described multi-layer crosslinked gelatin gel structure. According to the present invention, there is provided a preparation for release of a bioactive factor with the bioactive factor supported on the above-described multi-layer crosslinked gelatin gel structure.

According to the present invention, there is also provided a process for producing a multi-layer crosslinked gelatin gel structure, which comprises at least the following Steps 1 and 2:
Step 1 of applying an aqueous solution of gelatin or a gelatin derivative on to a support to form a coating layer, and then irradiating the coating layer with electron beam under an oxygen-containing atmosphere to form a first crosslinked gelatin gel layer; and
Step 2 of applying an aqueous solution of gelatin or a gelatin derivative on to the first crosslinked gelatin gel layer to form a coating layer, and then irradiating the coating layer with the electron beam under the oxygen-containing atmosphere to form a second crosslinked gelatin gel layer.

In order to prepare a multi-layer structure having 3 or more layers, there is adopted a process, in which the same step as the above-described Step 2 is further repeated desired times to successively form desired crosslinked gelatin gel layers on the second crosslinked gelatin gel layer.

According to the present invention, there is further provided a process for producing a preparation for release of a bioactive factor, which comprises supporting the bioactive factor on the above-described multi-layer crosslinked gelatin gel structure.

### BRIEF DESCRIPTION OF THE DRAWINGS

FIG. 1 is a graph illustrating an in vivo survival rate of crosslinked gelatin gel subcutaneously implanted into a mouse.
FIG. 2 is a graph illustrating a quantity of vascularization after 7 days from subcutaneous implantation of crosslinked gelatin gel with a bioactive factor (bFGF) supported thereon into a mouse.

### BEST MODE FOR CARRYING OUT THE INVENTION

Gelatin is mainly produced by using bovine bone, bovine skin and porcine skin as raw materials. A parent substance converted to gelatin among these raw materials is a protein called collagen. The collagen is a hardly soluble substance. However, when this collagen is pre-treated with an acid or alkali and then heated, a molecular structure of a triple-stranded helix is destroyed and divided into 3 random molecules. The collagen thermally denatured and solubilized in such a manner is called gelatin.

In order to extract high-quality gelatin from the collagen material, the material is pre-treated with an inorganic acid such as hydrochloric acid or sulfuric acid, or lime (alkali). The former and the latter are called acid-treated gelatin and alkali-treated gelatin, respectively, according to pretreatment conditions of the raw material.

Gelatin is an ampholyte. The ampholyte greatly changes a charged state depending on a pH of a solution. However, positive and negative charges in a molecule are balanced at a particular pH, and so the charges become zero as a whole. This pH is called an isoelectric point.

With respect to the amino acid composition of gelatin, it is reported that a proportion of amino acids having a polar group on a side chain is about 35%, and the details depending on the polar group thereof are as follows: a proportion of amino acids having a hydroxyl group is 15%, a proportion of amino acids having an acidic group is 12%, and a proportion of amino acids having a basic group is 8%.

In collagen of a raw material, one third of glutamic acid and asparagic acid each having an acidic group are amidated. However, an acid amide is hydrolyzed in a production process of gelatin and converted to a carboxyl group. The alkali-treated gelatin is deamidated by about 100% in a liming step, so that the isoelectric point thereof is as low as about 5. The deamidation rate of the acid-treated gelatin is low, so that the isoelectric point thereof is about 8 to 9.

No particular limitation is imposed on gelatin usable in the present invention so far as it can be crosslinked by irradiation of radiation and can support a bioactive factor, and a generally commercially available product may be used. For example, alkali-treated gelatin having an isoelectric point of 4.9 or 5.0 and acid-treated gelatin having an isoelectric point of 9.0, which are produced by Nitta Gelatin Inc. are representative thereof. These kinds of gelatin may be used singly. However, two or more kinds of gelatin, which are different in solubility, molecular weight, isoelectric point, raw material and the like, may be used in combination.

Bioactive factors (also referred to as "bioactive substances") are classified into a basic fibroblast growth factor (bFGF), a hepatocyte growth factor (HGF), a transforming growth factor (TGF-β1) and a bone morphogenetic protein factor (BMP-2), whose charge is cationic, and plasmid DNAs (plasmid DNAs coding the above-described growth factors), whose charge is anionic. When the bioactive factor is cationic, it is preferable to select anionic gelatin as the gelatin. When the bioactive factor is anionic, it is preferable to select cationic gelatin as the gelatin.

A gelatin derivative obtained by denaturing or modifying a side chain of gelatin may be used according to the kind of a bioactive factor supported thereon. Specific examples thereof include cationized gelatin derivatives obtained by graft-polymerizing ethylenediamine, spermidine or spermine on acid-treated gelatin having an isoelectric point of 9.0 with carbodiimide; and succinylated gelatin derivatives obtained by introducing succinic acid into a side chain of gelatin.

The multi-layer crosslinked gelatin gel structure according to the present invention can be produced by a production process comprising Step 1 of applying an aqueous solution of gelatin or a gelatin derivative on to a support to form a coating layer, and then irradiating the coating layer with electron beam under an oxygen-containing atmosphere to form a first crosslinked gelatin gel layer; and Step 2 of applying an aqueous solution of gelatin or a gelatin derivative on to the first crosslinked gelatin gel layer to form a coating layer, and then irradiating the coating layer with the electron beam under the oxygen-containing atmosphere to form a second crosslinked gelatin gel layer.

When the same step as Step 2 is further repeated desired times after Step 2 to successively form desired crosslinked gelatin gel layers on the second crosslinked gelatin gel layer, a multi-layer structure having desired layers and total thickness can be produced.

No limitation is imposed on the kind of the support and the coating method, and a continuous coating method or batch-wise coating method may be adopted. When the multi-layer crosslinked gelatin gel structure is produced by the batch-wise method, a process, in which an aqueous solution of gelatin or a gelatin derivative is cast in a casting mold (container) formed from glass, a metal or any one of various plastic materials to form a coating layer (cast layer), is preferred. The amount of the aqueous solution cast is controlled, whereby the film thickness of the coating layer can be exactly controlled. After the coating layer is irradiated with electron beam to crosslink gelatin, the next aqueous solution is cast on the resultant first crosslinked gelatin gel layer to form a coating layer, and the coating layer is irradiated with electron beam, whereby a second crosslinked gelatin gel layer can be formed. This process is successively repeated, whereby a multi-layer crosslinked gelatin gel structure having desired layers can be obtained.

A material of the casting mold or a material of the surface of the mold is desirably that undergoing no "repelling" when the aqueous solution of gelatin or gelatin derivative is cast. Examples of plastic materials for forming the casting mold include polystyrene, polypropylene, polyethylene and polyethylene terephthalate.

The concentration of the aqueous solution of gelatin or gelatin derivative is generally within a range of 1 to 90% by weight. The concentration of the aqueous solution of gelatin or gelatin derivative, which is used for forming each crosslinked gelatin gel layer making up the multi-layer structure, is within a range of generally 1 to 90% by weight, preferably 5 to 80% by weight, more preferably 5 to 50% by weight. With respective to the concentration of the aqueous solution of gelatin, however, the preferable range thereof varies according to the kind of the gelatin. In the case of, for example, high-molecular weight gelatin having an average molecular weight of 100,000 or higher, the solid content concentration (concentration of gelatin) of the aqueous solution is preferably 5 to 30% by weight because the viscosity of the solution is high. In the case of the gelatin derivative, the solid content concentration (concentration of gelatin derivative) of the aqueous solution is preferably 10 to 80% by weight because the viscosity of the solution is low.

If the solid content concentration of the aqueous solution of the gelatin or gelatin derivative is too thin, it is difficult to form a crosslinked gelatin gel layer having a sufficient crosslinking density, and the adhesion at an interface between the respective layers is also lowered. If the solid content concentration of the aqueous solution of the gelatin or gelatin derivative is too thick, it is difficult to form a coating layer (film) having a uniform thickness upon coating (casting). In addition, the efficiency of the crosslinking reaction by the irradiation of electron beam is lowered, and the interlayer separation easily occurs.

Since the coating layer formed of the aqueous solution of the gelatin or gelatin derivative is crosslinked in a state of hydrogel substantially without removing water, the thickness of the coating layer almost consists with the thickness of the resulting crosslinked gelatin gel layer.

The thickness of each coating layer varies according to the kind of the gelatin or gelatin derivative and the solid content concentration. However, 5 to 2,500 µm is generally proper as a range capable of forming a layer having a uniform film thickness. The thickness of each coating layer is preferably 10 to 2,000 µm, more preferably 30 to 1,500 µm, particularly preferably 50 to 1,000 µm from the viewpoints of easiness of the multi-layering operation and crosslinkability by the irradiation of electron beam.

In many cases, the thickness of each coating layer is controlled to about 70 to 500 µm, whereby the multi-layering operation is easily conducted, and a multi-layer crosslinked gelatin gel structure excellent in strength is easily obtained. The thickness of each coating layer is substantially retained even after the crosslinking by the irradiation of electron beam and substantially consists with the thickness of each crosslinked gelatin gel layer formed.

If the thickness of each coating layer is too thin, it takes a lot of time and labor to obtain a multi-layer crosslinked gelatin gel structure having a necessary thickness. If the thickness of each coating layer is too thick, uniform crosslinking by the irradiation of electron beam becomes difficult, interlayer adhesion is lowered, and the necessity of making the acceleration voltage excessively high arises.

No particular limitation is imposed on the total thickness of the multi-layer crosslinked gelatin gel structure according to the present invention, and the total thickness may be suitably set according to the form of the resulting preparation and the method of administration. In order to apply the preparation comprising the multi-layer crosslinked gelatin gel structure according to the present invention to an implant (implanting into a living body), the total thickness of the multi-layer crosslinked gelatin gel structure is desirably controlled to preferably 300 to 10,000 µm, more preferably 500 to 5,000 µm, particularly preferably 700 to 2,000 µm from the viewpoints of handling property and a period of gradual release.

If the total thickness of the multi-layer crosslinked gelatin gel structure is too thin, its strength becomes insufficient to deteriorate handling property upon implanting into a living body, and it is difficult to gain a necessary period of gradual release. If the total thickness of the multi-layer crosslinked gelatin gel structure is too thick, it takes a lot of time and labor to form the multi-layer structure, and irradiation energy is increased, so that the production cost becomes expensive.

The number of the crosslinked gelatin gel layers making up the multi-layer crosslinked gelatin gel structure may be optionally set according to the total thickness of the multi-layer structure and the thickness of each crosslinked gelatin gel layer. However, at least 2 layers are required. The layer structure is determined to be at least 2 layers, whereby the efficiency of the crosslinking by the irradiation of electron beam can be enhanced compared with the single-layer crosslinked gelatin gel, a multi-layer structure having a desired thickness can be provided, and the releasing rate of the bioactive factor can be controlled. The upper limit of the number of layers is generally about 100 layers, often about 50 layers or 30 layers. However, the upper limit is not limited thereto.

A commonly used electron beam irradiation apparatus may be used for irradiation of electron beam. The production of the crosslinked gelatin gel layers is restricted by the characteristics of the electron beam irradiation apparatus. For example, when an electron beam irradiation apparatus, whose acceleration voltage is 200 kV, is used to irradiate the coating layer of the gelatin or gelatin derivative with electron beam, a relative dose is attenuated to about 45% at a portion deep by 200 µm from the surface when an absorbed dose at the surface is regarded as 100%. When an electron beam irradiation apparatus, whose acceleration voltage is 800 kV, is used, a relative dose is attenuated to about 28% at a portion deep by 2,500 µm from the surface. The relation between the acceleration voltage and the attenuation of the relative dose is not proportional. When the relative dose at the deep portion is attenuated, the crosslinking effect at that portion by the irradiation of electron beam is also lowered. In order to conduct uniform crosslinking, it is desirable that the irradiation is conducted at such an acceleration voltage that a high relative dose is achieved, or the thickness of an object to be irradiated is controlled.

The exposure dose of the electron beam to each coating layer is preferably selected from a range of 5 to 20,000 kGy. The optimum value of the exposure dose of the electron beam greatly varies depending on the acceleration voltage and properties of the object to be irradiated. For example, at an acceleration voltage of 200 kV, the exposure dose of 10 to 20,000 kGy permits forming a good crosslinked gelatin gel layer. At an acceleration voltage of 800 kV, the exposure dose of 5 to 5,000 kGy permits forming a good crosslinked gelatin gel layer.

The exposure dose is related to the crosslinking density of the resulting crosslinked gelatin gel. The crosslinking density becomes high as the exposure dose is great. The exposure dose is preferably 15 to 3,000 kGy, more preferably 20 to 2,000 kGy when the acceleration voltage is 200 to 800 kV, and the thickness of the coating layer is of the order of 70 to 500 µm though it varies depending on the acceleration voltage and the thickness of the coating layer. However, when the thickness of each coating layer is great, the acceleration voltage may be raised, or the exposure dose may be increased. A relatively great exposure dose is preferably selected to a gelatin derivative obtained by adding a functional group having an electric or steric hindrance to a side chain of gelatin because the crosslinking efficiency is poor.

The irradiation of electron beam is generally conducted under an atmosphere of an inert gas such as nitrogen for the purpose of avoiding generation of ozone and increasing a reaction efficiency. As a result of researches by the present inventors, it has been proved that it is important from the viewpoint of enhancing the interlayer adhesion that the atmosphere, under which the coating layer (hydrogel) of the aqueous solution of the gelatin or gelatin derivative is irradiated with electron beam, is an oxygen-containing atmosphere, not an inert gas atmosphere.

The oxygen content in the oxygen-containing atmosphere is generally 1% by volume or more, preferably 5% by volume or more, more preferably 5 to 30% by volume. Other components thereof include inert gases such as nitrogen. In many cases, the irradiation of electron beam is conducted in air (the atmosphere) having an oxygen content of about 21% by volume, whereby the expected object can be achieved. A gas mixture of inert gases such as nitrogen gas and oxygen gas may also be used.

It is inferred that when the irradiation of electron beam is conducted under the oxygen-containing atmosphere, an oxygen molecule in the irradiation atmosphere is bonded to a molecule of the gelatin or gelatin derivative located on the surface portion of the coating layer to increase surface energy, thereby improving adhesion at an interface between the resultant crosslinked gelatin gel layer and a crosslinked gelatin gel layer newly provided on said crosslinked gelatin gel layer. A new crosslinked gelatin gel layer is formed on the lower crosslinked gelatin gel layer in a state that the surface energy on the lower crosslinked gelatin gel layer has been increased until a necessary thickness has been reached, thereby conducting the multi-layering of crosslinked gelatin gel layers.

In order to increase the surface energy of the crosslinked gelatin gel layer, additional irradiation is conducted while controlling the acceleration voltage so as not to change the crosslinking degree in the interior of the crosslinked gelatin gel layer after the crosslinking by the irradiation of electron beam, whereby only the surface portion can be treated. In other words, after the crosslinking treatment is conducted by the irradiation of electron beam at a high acceleration voltage, an additional irradiation treatment may be conducted at an acceleration voltage lower than that voltage. For example, when the acceleration voltage of 200 kV or higher is used in the crosslinking reaction, the irradiation of electron beam for the surface treatment may be conducted under such conditions that an electron current does not reach a deep portion, for example, the acceleration voltage is controlled to 100 kV. In other words, after the irradiation of electron beam is conducted at an acceleration voltage that an electron current reaches the deep portion of the coating layer to form a crosslinked gelatin gel layer, a step of irradiating with electron beam at an acceleration voltage that an electron current reaches only the surface portion of the crosslinked gelatin gel layer is added. Thereafter, a coating layer is formed on said crosslinked gelatin gel layer.

The respective crosslinked gelatin gel layers making up the multi-layer crosslinked gelatin gel structure may be composed of crosslinked gelatin gel layers different in crosslinking density from each other. For example, at least one surface portion of the multi-layer structure is formed of at least one fast degradable layer, i.e., at least one crosslinked gelatin gel layer low in crosslinking density, and at least one slowly degradable layer, i.e., at least one crosslinked gelatin gel layer high in crosslinking density, is provided as another layer (for example, a core layer), whereby it is possible to reconcile initial high-concentration gradual release of a bioactive factor supported on the multi-layer crosslinked gelatin gel structure with stable gradual release over a long period of time. It is difficult to directly measure the crosslinking density. However, the crosslinking degree may be evaluated by the exposure dose. The crosslinking density becomes high as the exposure dose is great.

Specific preferable examples include a method of controlling the exposure dose in such a manner that the exposure dose of electron beam to at least one crosslinked gelatin gel layer arranged at both surface portions becomes relatively smaller than the exposure dose of electron beam to at least one crosslinked gelatin gel layer arranged at a core portion.

When a biodegradable polymer layer is arranged as one outermost layer of the multi-layer structure, there is also a method of controlling the exposure dose in such a manner that the exposure dose of electron beam to at least one crosslinked gelatin gel layer arranged at the surface portion on the side opposed to the biodegradable polymer layer becomes relatively smaller than the exposure dose of electron beam to at least one crosslinked gelatin gel layer arranged at other portions.

As needed, a biodegradable polymer layer may be arranged as any one outermost layer or an intermediate layer in the multi-layer crosslinked gelatin gel structure according to the present invention. For example, a film or sheet of a biodegradable polymer is placed in a casting mold in advance, and crosslinked gelatin gel layers are successively formed thereon, whereby a multi-layer crosslinked gelatin gel structure with the biodegradable polymer layer arranged as one outermost layer can be obtained. A solution of the biodegradable polymer may be cast in the casting mold to form the biodegradable polymer layer.

A preparation obtained by using, as a carrier, the multi-layer crosslinked gelatin gel structure with the biodegradable polymer layer arranged as one outermost layer permits a bioactive factor impregnated to be gradually released only in a desired direction. The preparation having such a layer structure can have a function of tissue regeneration and repair, or treatment and cure of an inflammation part by the gradual release of the bioactive factor, and a function of prevention of coalescence between vital tissues.

The multi-layer crosslinked gelatin gel structure with the biodegradable polymer layer arranged as an intermediate layer is excellent in strength and hence excellent in handling property. Therefore, a preparation obtained by using, as a carrier, this multi-layer crosslinked gelatin gel structure can inhibit disintegration or interlayer separation of the multi-layer crosslinked gelatin gel structure when implanted into a movable part in a living body to retain stable release of the bioactive factor.

When a strand-like multi-layer crosslinked gelatin gel structure formed by successively coating a fibrous core material formed from the biodegradable polymer with crosslinked gelatin gel layers is impregnated with a bioactive factor, such a multi-layer structure can be used as a suture having an inflammation-curing effect.

Examples of the biodegradable polymer include aliphatic polyester resins such as polylactic acid (polylactide), polyglycolic acid (polyglycolide), polylactone and copolymers of respective monomers forming these polymers. Polycaprolactone is representative of the polylactone.

Since the biodegradable polymer has compatibility with a living body and is degraded and absorbed in the living body, the polymer is a material suitable for use in combination with the multi-layer crosslinked gelatin gel structure. The biodegradable polymer layer may be subjected to a corona discharge treatment or electron beam irradiation treatment for enhancing the interlayer adhesion between the biodegradable polymer layer and the crosslinked gelatin gel layer.

The thickness of the biodegradable polymer layer is generally 5 to 2,500 µm, preferably 8 to 2,000 µm, more preferably 10 to 1,000 µm. However, the thickness is not limited to these ranges. The form of the biodegradable polymer layer may be in any form of a film, a sheet, a net, a particle and a fiber.

No particular limitation is imposed on the form of the multi-layer crosslinked gelatin gel structure, and the multi-layer structure may be shaped into any form, for example, a column, prism, sheet, disk, sphere or particle. The multi-layer crosslinked gelatin gel structure with a bioactive factor supported thereon is generally used in a system implanted into a living body by a surgical operation. However, the multi-layer structure in the particulate form may be administered by injection.

A specific production example of the multi-layer crosslinked gelatin gel structure will be described. An aqueous solution of gelatin or a gelatin derivative is first adjusted to a temperature of generally 15 to 50°C, preferably 20 to 45°C, more preferably 25 to 43°C. If the temperature of the aqueous solution is too low, it is difficult to conduct coating (casting). If the temperature is too high, volatilization of water becomes great, and it is difficult to retain a desired concentration. The aqueous solution of the gelatin or gelatin derivative is then cast in a volume to give a desired thickness into a casting mold having a predetermined form.

As examples of the irradiation of electron beam, may be mentioned irradiation at an upper-limit acceleration voltage of 200 kV using an electron beam irradiation apparatus "CURETRON EBC-200-20-15" manufactured by NHV Corporation and irradiation at an upper-limit acceleration voltage of 800 kV using an electron beam irradiation apparatus "ESP-800" manufactured by NHV Corporation. However, the present invention is not limited to these apparatus and acceleration voltages.

Upon the irradiation of electron beam, a coating layer is irradiated with electron beam in a desired exposure dose (kGy) defined by an electron current (mA) and a travel speed (m/min) of an object to be irradiated in an irradiation zone to crosslink the gelatin or gelatin derivative. An aqueous solution of gelatin or a gelatin derivative is cast on the crosslinked gelatin gel layer thus formed in the same manner as described above and then irradiated with electron beam to crosslink the gelatin or gelatin derivative. Such a process is repeated, whereby a multi-layer crosslinked gelatin gel structure having desired layers and total thickness can be obtained.

As described above, prior to the formation of a new coating layer on the previously formed crosslinked gelatin gel layer, a step of irradiating only the vicinity of the surface of the crosslinked gelatin gel layer, which will become a lower layer, with electron beam for increasing surface energy is preferably performed at a low acceleration voltage (for example, 100 kV). The irradiation of electron beam is conducted under an oxygen-containing atmosphere, whereby adjoining respective crosslinked gelatin gel layers can be strongly bonded to each other. As a result, the multi-layer crosslinked gelatin gel structure can be prevented from causing interlayer separation when swollen with a drug liquid.

The bioactive factor supported on the crosslinked gelatin gel can be freely selected within the limits of the purpose of being implanted or administered into a living body to gradually release it. As a typical example of the bioactive factor, may be mentioned a basic fibroblast growth factor (bFGF). bFGF is a substance proved to stimulate cell proliferation for many kinds of cells such as vascular endothelial cell, vascular smooth muscle cell, corneal endothelial cell, osteoblast and chondrocyte as well as fibroblast.

Other examples of the bioactive factor include a transforming growth factor (TGF-β1), a hepatocyte growth factor (HGF), a platelet-derived growth factor (PDGF-BB), a keratinocyte growth factor (KGF), a bone morphogenetic protein (BMP-2), a vascular endothelial growth factor (VEGF) and plasmid DNAs coding these factors.

Examples of the bioactive factor also include anticancer drugs (adriamycin), angiotensin II receptor antagonists [TELMISARTAN: MICARDIS (Boehringer Ingelheim), CANDESARTAN (Takeda Pharmaceutical Company Limited), VALSARTAN (Novartis Pharma K.K.)] for protection of organs and lowering of blood pressure, and erythropoietin (protein preparation, hematopoietic hormone).

One or more factors may be selected from these bioactive factors as needed. The bioactive factor is generally supported on the multi-layer crosslinked gelatin gel structure by impregnating it into the multi-layer crosslinked gelatin gel structure.

The multi-layer crosslinked gelatin gel structure obtained in the above-described manner can be dried under reduced pressure or freeze-dried. A drug liquid (solution of a bioactive factor) may be impregnated even in a state of crosslinked gelatin hydrogel. However, the drug liquid can be rapidly impregnated by providing a freeze-dried structure or dried structure.

As an example of freeze-drying, may be mentioned a process, in which after the multi-layer crosslinked gelatin gel structure is frozen under conditions that the structure is held for at least 30 minutes in liquid nitrogen or for at least one hour in an extremely low-temperature freezer of from -90°C to -80°C, the frozen structure is preserved at -40°C in a square-type drying chamber (DRC-1000, manufactured by TOKYO RIKAKIKAI CO., LTD.) and then dried for 1 to 3 days at -10°C with a pressure reduced to 1 Pa or lower by a freeze dryer (EDU-2100, manufactured by TOKYO RIKAKIKAI CO., LTD.), and a step of temperature adjustment to 30°C is taken so as not to cause dew condensation upon taken out, thereby providing a freeze-dried structure. The multi-layer crosslinked gelatin gel structure according to the present invention is provided as a freeze-dried structure, whereby the storage stability thereof is improved in addition to drug liquid impregnating ability.

The multi-layer crosslinked gelatin gel structure according to the present invention has a layer structure that plural layers of crosslinked gelatin gel crosslinked by irradiating gelatin or a gelatin derivative with electron beam under an oxygen-containing atmosphere are arranged adjoiningly to each other. However, a biodegradable polymer layer may be arranged as at least one outermost layer or intermediate layer.

The multi-layer crosslinked gelatin gel structure according to the present invention is excellent in interlayer adhesion. As described in detail in Examples, the multi-layer crosslinked gelatin gel structure according to the present invention does not cause interlayer separation when a sample cut out of the structure into the size of 5 mm long and 5 mm wide so as to expose the sections of the respective layers to the cut surface in a thickness-wise direction is immersed for 24 hours in physiological saline at 25°C and then shaken and stirred for 10 seconds at 2,500 rpm by means of a test tube mixer.

The multi-layer crosslinked gelatin gel structure according to the present invention is provided as hydrogel (crosslinked hydrogel) at the time of production and may be converted to a freeze-dried structure by freeze-drying. The multi-layer structure may be dried by another drying method than the freeze-drying.

The multi-layer crosslinked gelatin gel structure according to the present invention may comprise a plurality of crosslinked gelatin gel layers different in exposure dose of electron beam from each other. The crosslinking density of the crosslinked gelatin gel layer varies according to the exposure dose. As a result, a concentration gradient with time can be imparted to the gradual releasability of the bioactive factor supported on the multi-layer crosslinked gelatin gel structure.

The multi-layer crosslinked gelatin gel structure is a suitable for use as a carrier for a bioactive factor. The bioactive factor is supported on the multi-layer crosslinked gelatin gel structure according to the present invention, whereby a preparation for release of the bioactive factor can be obtained. The bioactive factor is generally impregnated in the form of an aqueous solution into the multi-layer crosslinked gelatin gel structure. The content of the bioactive factor may be suitably set according to the kind and purpose thereof, desired quantity to be released and period of gradual release, etc.

### EFFECTS OF THE INVENTION

According to the present invention, there can be provided multi-layer crosslinked gelatin gel structures excellent in safety for living bodies and useful as carriers for bioactive factors.

The multi-layer crosslinked gelatin gel structures according to the present invention are excellent in safety for living bodies and can have a desired thickness because the crosslinking process by irradiation of electron beam is adopted, and the structure is multi-layered. The multi-layer crosslinked gelatin gel structures are excellent in transparency because the crosslinking process by irradiation of electron beam is adopted.

The exposure dose of the electron beam is changed to vary the crosslinking densities of the respective crosslinked gelatin gel layers, whereby a multi-layer crosslinked gelatin gel structure, by which the degradation speed in a living body can be optionally controlled, can be provided.

When a bioactive factor is supported on such a multi-layer crosslinked gelatin gel structure, a preparation for release of the bioactive factor, by which change of concentration with time can be given in the release of the bioactive factor, can be provided.

The preparation for release of the bioactive factor according to the present invention can adjust the period of gradual release of the bioactive factor after implanted into a living body to about 3 days to 5 weeks and also control increase or decrease in the quantity to be gradually released. The total thickness, exposure dose and the like are controlled, whereby a preparation, which can gradually release the bioactive factor over a longer period, can also be produced. The multi-layer crosslinked gelatin gel structures according to the present invention are excellent in handling property because interlayer separation is hard to occur upon the impregnation of the aqueous solution of the bioactive factor.

When the multi-layer crosslinked gelatin gel structure with the biodegradable polymer layer arranged as one outermost layer is used as a carrier, a preparation, which permits, for example, curing inflammation in a living body by the release of the bioactive factor and preventing coalescence in a vital tissue at the same time, can be provided. When the biodegradable polymer layer is arranged as an intermediate layer of the multi-layer crosslinked gelatin gel structure, a multi-layer crosslinked gelatin gel structure having high strength can be provided.

### EXAMPLES

The present invention will hereinafter be described more specifically by Examples, Comparative Examples and Test Examples. However, the present invention is not limited to these examples.

### Example 1

An aqueous solution (concentration: 10% by weight) of alkali-treated gelatin (product of Nitta Gelatin Inc.; isoelectric point: 5.0) of I-type collagen derived from bovine bone was cast on an inner bottom surface of a casting mold (polystyrene container; 86 mm in diameter x 12 mm in depth) to form a uniform coating layer having a thickness of 100 µm. Substantially without removing water in the coating layer, the coating layer was then irradiated with electron beam at an acceleration voltage of 200 kV under an air atmosphere by means of an electron beam irradiation system "CURETRON EBC200-20-15" (manufactured by NHV Corporation) so as to give an exposure dose of 60 kGy. A first crosslinked gelatin gel layer was formed in this manner.

The above-described aqueous solution of the alkali-treated gelatin was cast on this first crosslinked gelatin gel layer to form a uniform coating layer having a thickness of 100 µm, and the coating layer was irradiated with electron beam under the same conditions as described above. A second crosslinked gelatin gel layer was provided on the first crosslinked gelatin gel layer in this manner. This process was repeated further 8 times (10 times in total) to produce a multi-layer crosslinked gelatin gel structure having a total thickness of 1,000 µm. This multi-layer crosslinked gelatin gel structure was taken out of the casting mold. The multi-layer crosslinked gelatin gel structure was hydrogel. The process conditions and results are shown in Table 1.

### Example 2

After the multi-layer crosslinked gelatin gel structure produced in Example 1 was frozen for 24 hours in an extremely low-temperature freezer ("MDF-U481ART", manufactured by SANYO Electric Co., Ltd.) of -85°C, the frozen multi-layer crosslinked gelatin gel structure was preserved at -40°C in a square-type drying chamber (DRC-1000, manufactured by TOKYO RIKAKIKAI CO., LTD.) and then dried for 3 days at -10°C with a pressure reduced to 1 Pa or lower by a freeze dryer (EDU-2100, manufactured by TOKYO RIKAKIKAI CO., LTD.), and a step of temperature adjustment to 30°C was taken so as not to cause dew condensation upon taken out, thereby providing a freeze-dried structure. The process conditions and results are shown in Table 1.

### Example 3

An aqueous solution (concentration: 10% by weight) of alkali-treated gelatin (product of Nitta Gelatin inc.; isoelectric point: 5.0) of I-type collagen derived from bovine bone was cast in a casting mold to form a uniform coating layer having a thickness of 200 µm. Substantially without removing water in the coating layer, the coating layer was then irradiated with electron beam at an acceleration voltage of 200 kV under an air atmosphere by means of an electron beam irradiation system "CURETRON EBC200-20-15" (manufactured by NHV Corporation) so as to give an exposure dose of 60 kGy. A first crosslinked gelatin gel layer was formed in this manner. The above-described aqueous solution of the alkali-treated gelatin was cast on this first gelatin gel layer to form a uniform coating layer having a thickness of 200 µm, and the coating layer was irradiated with electron beam under the same conditions as described above. A second crosslinked gelatin gel layer was formed in this manner. This process was repeated further 3 times (5 times in total) to produce a multi-layer crosslinked gelatin gel structure having a total thickness of 1,000 µm. The process conditions and results are shown in Table 1.

### Example 4

The process was performed in the same manner as in Example 1 except that the exposure dose of the electron beam was changed from 60 kGy to 20 kGy, thereby producing a multi-layer crosslinked gelatin gel structure having a total thickness of 1,000 µm. The process conditions and results are shown in Table 1.

### Example 5

The process was performed in the same manner as in Example 1 except that acid-treated gelatin (product of Nitta Gelatin Inc.; isoelectric point: 9.0) of I-type collagen derived from porcine skin was used in place of the alkali-treated gelatin in Example 1, thereby producing a multi-layer crosslinked gelatin gel structure having a total thickness of 1,000 µm. The process conditions and results are shown in Table 1.

### Example 6

An aqueous solution (concentration: 20% by weight) of a cationized gelatin derivative (a material obtained by grafting ethylenediamine on acid-treated gelatin having an isoelectric point of 9.0 with carbodiimide; product of NICHIBAN CO., LTD.) was cast in a casting mold to form a uniform coating layer having a thickness of 100 µm. Substantially without removing water in the coating layer, the coating layer was irradiated with electron beam at an acceleration voltage of 200 kV under an air atmosphere by means of an electron beam irradiation system "CURETRON EBC200-20-15" (manufactured by NHV Corporation) so as to give an exposure dose of 300 kGy. A first crosslinked gelatin gel layer was formed in this manner. The same aqueous solution as described above was cast on this first crosslinked gelatin gel layer to form a uniform coating layer having a thickness of 100 µm, and the coating layer was irradiated with electron beam under the same conditions as described above. A second crosslinked gelatin gel layer was formed in this manner. This process was repeated further 8 times (10 times in total) to produce a multi-layer crosslinked gelatin gel structure having a total thickness of 1,000 µm. The process conditions and results are shown in Table 1.

### Example 7

In Example 1, the exposure dose of the electron beam upon the formation of all the 10 crosslinked gelatin gel layers was controlled to 60 kGy. This exposure dose was changed as follows. A multi-layer crosslinked gelatin gel structure having a total thickness of 1,000 µm was produced in the same manner as in Example 1 except that the exposure dose to the first to third crosslinked gelatin gel layers in Example 1 was changed to 20 kGy, the exposure dose to the fourth to seventh crosslinked gelatin gel layers was controlled to 60 kGy, and the exposure dose to the eighth to tenth crosslinked gelatin gel layers was changed to 20 kGy. The process conditions and results are shown in Table 1.

### Comparative Example 1

An aqueous solution (concentration: 10% by weight) of alkali-treated gelatin (product of Nitta Gelatin Inc.; isoelectric point: 5.0) of I-type collagen derived from bovine bone was cast in a casting mold to form a uniform coating layer having a thickness of 1,000 µm. Substantially without removing water in the coating layer, the coating layer was then irradiated with electron beam at an acceleration voltage of 800 kV under an air atmosphere by means of an electron beam irradiation system "CURETRON EBC200-20-15" (manufactured by NHV Corporation) so as to give an exposure dose of 20 kGy. A single-layer crosslinked gelatin gel structure was formed in this manner. The process conditions and results are shown in Table 1.

### Comparative Example 2

An aqueous solution (concentration: 5% by weight) of alkali-treated gelatin (product of Nitta Gelatin Inc.; isoelectric point: 5.0) of I-type collagen derived from bovine bone was cast in a casting mold to form a uniform coating layer having a thickness of 200 µm. Substantially without removing water in the coating layer, the coating layer was then irradiated with electron beam at an acceleration voltage of 200 kV under a nitrogen gas atmosphere by means of an electron beam irradiation system "CURETRON EBC200-20-15" (manufactured by NHV Corporation) so as to give an exposure dose of 20 kGy. A first crosslinked gelatin gel layer was formed in this manner.

The above-described aqueous solution of the alkali-treated gelatin was cast on this first crosslinked gelatin gel layer to form a uniform coating layer having a thickness of 200 µm, and the coating layer was irradiated with electron beam under the same conditions as described above. A second crosslinked gelatin gel layer was formed in this manner. This process was repeated further 3 times (5 times in total) to produce a multi-layer crosslinked gelatin gel structure having a total thickness of 1,000 µm. The process conditions and results are shown in Table 1.

Test Example 1

### Interlayer adhesion test:

Each of the multi-layer crosslinked gelatin gel structures produced in Examples 1 to 7 and Comparative Example 2 was cut into the size of 5 mm long and 5 mm wide so as to expose the sections of the respective layers to the cut surface in a thickness-wise direction, thereby preparing a sample. This sample was immersed for 24 hours at 25°C in 3ml of physiological saline in a 15 ml cylindrical container to cause the physiological saline to be absorbed therein. Thereafter, shaking and stirring were conducted for 10 seconds at 2,500 rpm by means of a test tube mixer ("TTM-1", manufactured by SIBATA TECHNOLOGY Ltd.).

The sample of Comparative Example 2 prepared by conducting the irradiation of electron beam under the nitrogen gas atmosphere caused interlayer separation and separated into several layers. On the other hand, all the respective samples of Examples 1 to 7 prepared by conducting the irradiation of electron beam under the air atmosphere did not cause interlayer separation and retained the integral structure as the multi-layer structure. The results are shown in Table 1.

**Table 1**

| | | Gelatin | Concentration of aqueous solution (% by weight) | Thickness of each layer x number of layers [µ] x [number of layers] | Irradiation treatment with electron beam | | | Form of preparation | Condition at the time water was contained |
|---|---|---|---|---|---|---|---|---|---|
| | | | | | Acceleration voltage [kV] | Exposure dose [kGy] | Atmosphere | | |
| Example | 1 | IP = 5.0 alkali-treated | 10 | 100 x 10 | 200 | 60 | Air | Hydrogel | Good |
| | 2 | IP = 5.0 Alkali-treated | 10 | 100 x 10 | 200 | 60 | Air | Freeze-dried structure | Good |
| | 3 | IP = 5.0 Alkali-treated | 10 | 200 x 5 | 200 | 60 | Air | Hydrogel | Good |
| | 4 | IP = 5.0 Alkali-treated | 10 | 100 x 10 | 200 | 20 | Air | Hydrogel | Good |
| | 5 | IP = 9.0 Acid-treated | 10 | 100 x 10 | 200 | 100 | Air | Hydrogel | Good |
| | 6 | Cationic group-introduced gelatin derivative | 20 | 100 x 10 | 200 | 300 | Air | Hydrogel | Good |
| | 7 | IP = 5.0 Alkali-treated | 10 | 100 x 3 | 200 | 20 60 20 | Air | Hydrogel | Good |
| | | | | 100 x 4 | | | | | |
| | | | | 100 x 3; | | | | | |
| | | | | 100 x 10 | | | | | |
| | | | | in total | | | | | |
| Comp. Example | 1 | Alkali-treated | 10 | 1000 | 800 | 20 | Air | Hydrogel | Good |
| | 2 | IP = 5.0 Alkali-treated | 5 | 200 x 5 | 200 | 20 | Nitrogen | Hydrogel | Interlaver separation |

### Test Example 2

### Animal Experiment-Confirmation test of in vivo degradability:

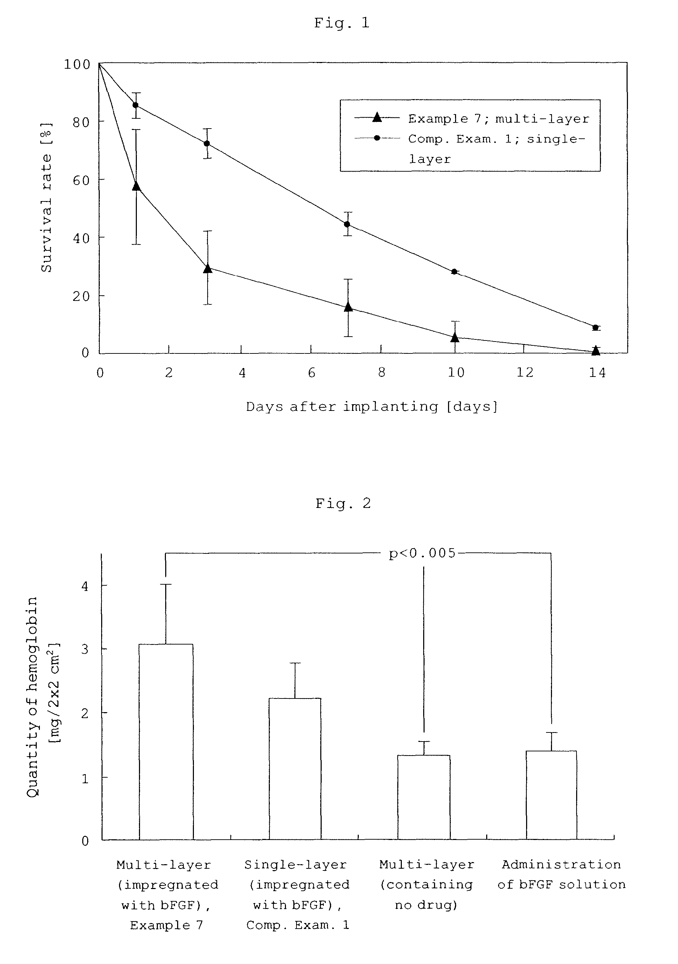

A sample (5 mm long x 5 mm wide x 1,000 µm thick) cut out of the multi-layer crosslinked gelatin gel structure prepared in Example 7 was subcutaneously implanted into a mouse (under the skin at the back thereof). Likewise, a sample (5 mm long x 5 mm wide x 1,000 µm thick) cut out of the single-layer crosslinked gelatin gel structure prepared in Comparative Example 1 was subcutaneously implanted into a mouse. With respect to these samples, in vivo degradability profile was confirmed. More specifically, the sample implanted was taken out after 1 day, 3 days, 7 days, 10 days and 14 days from the implantation, and the weight thereof was compared with the weight of the sample before the implantation to calculate out a survival rate. The results are illustrated in FIG. 1.

In the degradability profile, linear curve-like degradability was exhibited in the case of the single-layer crosslinked gelatin gel, whereas the multi-layer crosslinked gelatin gel structure is degraded in plenty just after the implantation. It is inferred that the degradation speed is parallel with the released quantity of the bioactive factor supported. The multi-layer crosslinked gelatin gel structure prepared in Example 7 contains a plurality of the crosslinked gelatin gel layers different in the exposure dose of electron beam from each other and releases the bioactive factor at a high concentration from the crosslinked gelatin gel layers small in the exposure dose (low in the crosslinking density) and high in the degradation speed in the initial stage of the implantation. It was confirmed that the multi-layer crosslinked gelatin gel structure can gradually release the bioactive factor for the period of the same days as the single-layer crosslinked gelatin gel.

### Test Example 3

### Animal Experiment-Confirmation test of pharmacological effect:

A sample (5 mm long x 5 mm wide x 1,000 µm thick) cut out of the multi-layer crosslinked gelatin gel structure prepared in Example 7 was impregnated with an aqueous solution containing 100 µg of basic fibroblast growth factor (bFGF). A preparation obtained in this manner was subcutaneously implanted into a mouse. Likewise, a sample (5 mm long x 5 mm wide x 1,000 µm thick) cut out of the single-layer crosslinked gelatin gel structure prepared in Comparative Example 1 was impregnated with the bFGF-containing aqueous solution to prepare a preparation, and the preparation was then subcutaneously implanted into a mouse. As a control, a sample of the multi-layer crosslinked gelatin gel structure (Example 7), into which the aqueous solution of bFGF was not impregnated, was subcutaneously implanted into a mouse. As a control likewise, the aqueous solution of bFGF (bFGF = 100 µg) was subcutaneously administered to a mouse by an injector.

A quantity of vascularization after 7 days from the implantation was measured as a quantity of hemoglobin per unit tissue. The results are illustrated in FIG. 2. The multi-layer crosslinked gelatin gel structure prepared in Example 7 contains a plurality of the crosslinked gelatin gel layers different in the exposure dose of electron beam from each other, releases the bioactive factor at a high concentration from the crosslinked gelatin gel layers small in the exposure dose (low in the crosslinking density) and high in the degradation speed in the initial stage of the implantation and is also excellent in long-term gradual releasability. It is understood from the results illustrated in FIG. 2 that the preparation for release of bFGF making use of the multi-layer crosslinked gelatin gel structure as a carrier exhibits an excellent vascularizing effect.

### Test Example 4

### Confirmation test of period allowing gradual release of bioactive factor:

(1) A multi-layer crosslinked gelatin gel structure composed of 5 crosslinked gelatin gel layers each having a thickness of 200 µm and having a total thickness of 1,000 µm was prepared in the same manner as in Example 3 except that the exposure dose of electron beam was changed to 20 kGy from 60 kGy in Example 3. An aqueous solution (concentration: 10% by weight) of alkali-treated gelatin (product of Nitta Gelatin Inc.; isoelectric point: 5.0) of I-type collagen derived from bovine bone was used as an aqueous solution of gelatin like Example 3. A sample (5 mm long x 5 mm wide x 1,000 µm thick) cut out of this multi-layer crosslinked gelatin gel structure vanished in 3 days after subcutaneously implanted into a mouse. Accordingly, this multi-layer crosslinked gelatin gel structure can gradually release a bioactive factor for 3 days.
(2) An aqueous solution (concentration: 10% by weight) of alkali-treated gelatin (product of Nitta Gelatin Inc.; isoelectric point: 5.0) of I-type collagen derived from bovine bone was cast in a casting mold to form a uniform coating layer having a thickness of 500 µm. Substantially without removing water in the coating layer, the coating layer was then irradiated with electron beam at an acceleration voltage of 800 kV under an air atmosphere by means of an electron beam irradiation system "CURETRON EBC200-20-15" (manufactured by NHV Corporation) so as to give an exposure dose of 50 kGy. A first crosslinked gelatin gel layer was formed in this manner.
   The above-described aqueous solution of the alkali-treated gelatin was cast on this first gelatin gel layer to form a uniform coating layer having a thickness of 500 µm, and the coating layer was irradiated with electron beam under the same conditions as described above. In this manner, a multi-layer crosslinked gelatin gel structure composed of 2 layers and having a total thickness of 1,000 µm, in which a second crosslinked gelatin gel layer was provided on the first crosslinked gelatin gel layer, was prepared.
   It was confirmed that 46.9% of a sample (5 mm long x 5 mm wide x 1,000 µm thick) cut out of this multi-layer crosslinked gelatin gel structure remains in the twenty-first day after subcutaneously implanted into a mouse. Accordingly, it is inferred that it takes about 44 days to completely degrade the multi-layer crosslinked gelatin gel structure in a living body, and so it was confirmed that a multi-layer crosslinked gelatin gel structure having degradability of 5 weeks (35 days) can be prepared.
(3) From the results of the above-described experiments, it was proved that the period of gradual release of a bioactive factor in the multi-layer crosslinked gelatin gel structure according to the present invention can be adjusted within a period of from 3 days to 5 weeks.

### INDUSTRIAL APPLICABILITY

The multi-layer crosslinked gelatin gel structures according to the present invention are excellent in safety for living bodies and can be used as carriers for bioactive factors.

When a bioactive factor is supported on the multi-layer crosslinked gelatin gel structure according to the present invention, the multi-layer crosslinked gelatin gel structure can be used as a preparation for release of the bioactive factor, by which change of concentration with time can be given in the release of the bioactive factor.

The preparation for release of the bioactive factor according to the present invention can gradually release the bioactive factor by administering it by, for example, implanting into a living body or injection using an injector.

## Claims

1. A multi-layer crosslinked gelatin gel structure having a layer structure that plural layers of crosslinked gelatin gel crosslinked by irradiating gelatin or a gelatin derivative with electron beam under an oxygen-containing atmosphere are arranged adjoiningly to each other.

2. The multi-layer crosslinked gelatin gel structure according to claim 1, which does not cause interlayer separation when a sample cut out of the structure into the size of 5 mm long and 5 mm wide so as to expose the sections of the respective layers to the cut surface in a thickness-wise direction is immersed for 24 hours in physiological saline at 25°C and then shaken and stirred for 10 seconds at 2,500 rpm by means of a test tube mixer.

3. The multi-layer crosslinked gelatin gel structure according to claim 1, which is hydrogel or a freeze-dried structure.

4. The multi-layer crosslinked gelatin gel structure according to claim 1, which comprises a plurality of crosslinked gelatin gel layers relatively different in exposure dose of the electron beam from each other.

5. The multi-layer crosslinked gelatin gel structure according to claim 4, wherein the exposure dose of the electron beam to at least one crosslinked gelatin gel layer arranged at one or both surface portions is relatively smaller than the exposure dose of the electron beam to at least one crosslinked gelatin gel layer arranged at other portions.

6. The multi-layer crosslinked gelatin gel structure according to claim 1, wherein the thickness of each of the crosslinked gelatin gel layers is 5 to 2,500 µm, and the total thickness of the crosslinked gelatin gel layers is 300 to 10,000 µm.

7. The multi-layer crosslinked gelatin gel structure according to claim 1, wherein a biodegradable polymer layer is additionally arranged as any one outermost layer or an intermediate layer.

8. A carrier for a bioactive factor, comprising the multi-layer crosslinked gelatin gel structure according to any one of claims 1 to 7.

9. A preparation for release of a bioactive factor with the bioactive factor supported on the multi-layer crosslinked gelatin gel structure according to any one of claims 1 to 7.

10. The preparation for release of the bioactive factor according to claim 9, wherein the bioactive factor is at least one bioactive factor selected from the group consisting of a basic fibroblast growth factor (bFGF), a transforming growth factor (TGF-β1), a hepatocyte growth factor (HGF), a platelet-derived growth factor (PDGF-BB), a keratinocyte growth factor (KGF), a bone morphogenetic protein (BMP-2), a vascular endothelial growth factor (VEGF), plasmid DNAs coding these growth factors, an anticancer drug, an angiotensin II receptor antagonist, and a protein preparation.

11. A process for producing a multi-layer
crosslinked gelatin gel structure, which comprises at least the following Steps 1 and 2:
Step 1 of applying an aqueous solution of gelatin or a gelatin derivative on to a support to form a coating layer, and then irradiating the coating layer with electron beam under an oxygen-containing atmosphere to form a first crosslinked gelatin gel layer; and
Step 2 of applying an aqueous solution of gelatin or a gelatin derivative on to the first crosslinked gelatin gel layer to form a coating layer, and then irradiating the coating layer with the electron beam under the oxygen-containing atmosphere to form a second crosslinked gelatin gel layer.

12. The production process according to claim 11, which further comprises a step of repeating the same step as Step 2 desired times after Step 2 to successively form desired crosslinked gelatin gel layers on the second crosslinked gelatin gel layer.

13. The production process according to claim 11, wherein the support is a casting mold, the coating layer formed of the aqueous solution of the gelatin or gelatin derivative in each step is formed by casting of the aqueous solution in the casting mold.

14. The production process according to claim 11, wherein the solid content concentration of the aqueous solution of the gelatin or gelatin derivative is 1 to 90% by weight.

15. The production process according to claim 11, wherein the thickness of each of the crosslinked gelatin gel layers is 5 to 2,500 µm, and the total thickness of the crosslinked gelatin gel layers is 300 to 10,000 µm.

16. The production process according to claim 11, wherein the exposure dose of the electron beam to each crosslinked gelatin gel layer is 5 to 20,000 kGy.

17. The production process according to claim 11, wherein the exposure dose of the electron beam to the respective coating layers is changed to form a plurality of crosslinked gelatin gel layers relatively different in the exposure dose of the electron beam from each other.

18. The production process according to claim 11, which comprises an additional step of arranging a biodegradable polymer layer as any one outermost layer or an intermediate layer.

19. The production process according to claim 11, wherein a step of freeze-drying the multi-layer crosslinked gelatin gel structure is further arranged.

20. A process for producing a preparation for release of a bioactive factor, which comprises supporting the bioactive factor on the multi-layer crosslinked gelatin gel structure obtained by the production process according to any one of claims 11 to 19.
